# EUROPEAN PATENT SPECIFICATION

(11) **EP 2 799 618 B1**
(45) Date of publication and mention of the grant of the patent: **27.04.2016**
(21) Application number: 13165746.2
(22) Date of filing: 29.04.2013
(51) Int. Cl.: D21H 21/30, C07D 251/00, C09K 11/07

(54) **Use of micronized cellulose and fluorescent whitening agent for surface treatment of cellulosic materials**
Verwendung von mikronisierter Cellulose und fluoreszierendes Weißmittel zur Oberflächenbehandlung von Cellulosematerialien
Utilisation de cellulose micronisée et agent de blanchiment fluorescent pour traitement de surface de matériaux cellulosiques

(43) Date of publication of application: 05.11.2014
(73) Proprietor: Blankophor GmbH & Co. KG, 49577 Ankum (DE)
(72) Inventor: Hauschel, Bernd, 51467 Bergisch Gladbach (DE); Kraemer, Michael, 51515 Kürten (DE); Hunke, Bernhard, 53773 Hennef (DE); Klug, Günter, 40764 Langenfeld (DE)
(74) Representative: Lang, Johannes

(56) References cited:
- EP-A1- 0 409 028
- EP-A1- 2 431 519
- WO-A1-01/19804
- WO-A1-2006/045714
- WO-A1-2011/056130
- WO-A1-2011/064441
- WO-A1-2012/126628
- WO-A1-2012/171876

## Description

The present invention relates to the use of micronized cellulose and fluorescent whitening agents for surface treatment of cellulosic sheet-formed materials, preparations comprising micronized cellulose and fluorescent whitening agents, and a process for surface whitening of cellulosic sheet-formed materials using the preparations. These preparations enable improved fluorescent whitening and optical brightening, respectively, of cellulosic materials like paper, board or cotton fabric in advantageous manner.

The use of fluorescent whitening agents (FWA's), also called optical brighteners, for the whitening of cellulosic materials like paper, board and cotton fabric is well-known. Paper is essentially composed of cellulose fibers and mineral fillers, or precipitated fillers. Coated paper contains in addition one or several so-called coating layers as surface finish, those layers are essentially composed of inorganic white pigments and binding agents. The coating layers are typically applied in a separate processing step to the coating base paper, containing cellulose fibers and fillers.

It is common during papermaking to treat printing and writing paper, as well as coated packaging and board paper grades, with fluorescent whitening agents in order to improve their brightness and whiteness. There are several possibilities to add the fluorescent whitening agents during the papermaking process. The fluorescent whitening agents can be added before formation of the paper web to the paper stock or paper furnish, respectively, which is essentially a slurry of cellulose fibers in water that contains additionally inorganic fillers and, where necessary, process and functional chemicals. The consistency of the paper stock is typically below 5 weight-% of solid material at the point where the fluorescent whitening agents are added. As another option, the fluorescent whitening agents can be applied after the formation of the paper web to the paper surface. In case of uncoated paper, the application to the paper surface is carried out with the aid of a suitable surface treatment device, e.g. a size press or a film press. Typically, the fluorescent whitening agents are applied jointly with a starch solution, wherein this solution may contain further chemicals, e.g. surface sizing agents. In case of coated paper, the fluorescent whitening agents can be added to the coating color, which is an aqueous preparation essentially containing inorganic white pigments, binders and other additives. The coating color is applied to a coating base paper and thus coated paper is produced. The three mentioned basic kinds of applying fluorescent whitening agents may be used alone or in combination, depending on the particular paper grade and the individual whiteness target.

The brightening of paper by surface treatment is, inter alia, described in detail in the following article: *"*Surface brightening of Paper" from H. Weaver, in: Surface Application of Paper Chemicals, Eds. James Brandner and Ian Thorn, Blackie Academic & Professional, 1. Edition 1997, pages 156-174.

As fluorescent whitening agents for brightening and whitening of cellulosic materials, derivatives of 4,4'-diamino-2,2'-stilbenedisulfonic acid are known. Typical examples thereof are those in which 4,4'-diamino-2,2'-stilbenedisulfonic acid is substituted with two triazine rings which are bearing amino residues. From those fluorescent whitening agents disulfo-, tetrasulfo- and hexasulfo-types are known.

The whitening effect of 4,4'-diamino-2,2'-stilbenedisulfonic acid derivatives is caused by the fact that their *trans*-form absorbs light in the near UV-region, which brings about electronic excitation. The absorption maximum is at about 350 nm. The excited state may then be deactivated by emission of fluorescent light in the visible, blue spectral region with a maximum at about 420-450 nm. The emitted blue light leads to a compensation, or even over-compensation, of the natural yellowish tint of the cellulosic material. The resulting bluish white is perceived by the human eye as clearly *"whiter",* compared to the initial yellowish white of the untreated cellulosic material. The brightness and whiteness of such materials can be measured as ISO brightness and/or CIE whiteness according to known methods, to quantify the effect of a fluorescent whitening agent treatment.

In case of adding fluorescent whitening agents to the paper stock, the whitening effect is mainly dependent on the purity of the fluorescent whitening agent used and its adsorption rate to the cellulose fibers. The adsorption rate is mainly influenced by the selected point of fluorescent whitening agent addition to the paper stock, thus by the contact time between fluorescent whitening agent and papermaking fibers until paper web formation, and further by the solubility of the fluorescent whitening agent used. The adsorption rate is inversely correlated with the number of sulfo groups in the fluorescent whitening agent molecule in such a way that the more sulfo groups the fluorescent whitening agent molecule is bearing, the lower the adsorption rate is.

On the other hand, in case of surface treatment of uncoated paper and coating colors for coated paper production through use of fluorescent whitening agents, for achieving a good whitening effect it is important to avoid the aggregation or association of the fluorescent whitening agent molecules on the material to be treated. Such an association occurs e.g. in case of overdosing of fluorescent whitening agents leading to the so-called greening effect. It is known to minimize the association tendency and thus to shift the greening limit to higher amounts of fluorescent whitening agent by using either specific types of fluorescent whitening agents or by adding specific auxiliaries. Generally, the tendency to associate is diminished with increasing water solubility of the fluorescent whitening agent molecule. Thus, fluorescent whitening agents of the hexasulfo-type have generally a lower association tendency, compared to the corresponding fluorescent whitening agents of the tetrasulfo-type. Furthermore, the association tendency can be minimized by adding carrier polymers, which are hydrophilic, highly water soluble polymers, such as starches, linear polyethylene glycols with a medium molecular weight up to 8000 g/mol, or polyvinyl alcohol.

In the prior art, fluorescent whitening agents are used in form of aqueous solutions, slurries or dry powders. Before contact with paper fibers, slurries and dry powders of fluorescent whitening agents are dissolved in water so that they form aqueous solutions, in order to ensure an even distribution in the papermaking stock or on the paper surface as good as possible, and also to avoid undesired effects, for example fluorescent spots on the paper or veining.

WO 2011/064441 A1 discloses a method for producing cellulose pulp containing at least 30 weight-% of nanofibrillated cellulose (NFC) material. The manufacturing process involves the addition of optical brightening agents prior to or during the refining and/or fibrillation stage of the cellulose pulp, to improve the production efficiency of the nanofibrillated cellulose pulp. In the examples of WO 2011/064441, as refining additive 2 weight-% of optical brightening agent of disulfonic type or hexasulfonic type were dosed to the pulp before the pre-refiner stage.

WO 2011/056130 is directed to a coated substrate having a dispersion coating comprising microfibrillated cellulose (MFC) and colloidal particles of a polymer, to form a barrier on the surface of the substrate.

EP 0 409 028 A1 refers to a process for whitening paper coating compositions and whitener preparations for this process, using combinations of cellulose powder (average particle size below 50 µm) and anionic cellulose optical brighteners.

Surprisingly, it has been found that problems of the prior art can be overcome and the surface whitening of paper or other cellulosic materials can be improved by the combined use of micronized cellulose and fluorescent whitening agent in the surface treatment of the cellulosic materials. In particular, when using fluorescent whitening agent-containing particles of micronized cellulose for the surface whitening of uncoated paper, or for the whitening of coating colors, an outstanding whitening effect and improved whitening behavior compared to the prior art application is observed. The micronized cellulose has in particular a medium fiber length and medium particle size, respectively, below or equal to 15 µm.

Therefore, the present invention relates to the use of
(1) micronized cellulose having a medium fiber length and medium particle size, respectively, below or equal to 15 µm, having a maximum fiber diameter and maximum particle diameter, respectively, not exceeding 25 µm and being essentially free from fibers with a length exceeding 100 µm, and
(2) at least one fluorescent whitening agent of formula (1) 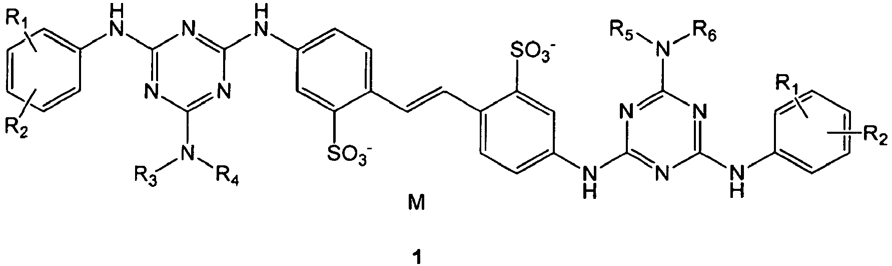 wherein
   R₁ and R₂ independently of each other mean H, SO₃⁻ or COOH, with the proviso that, if R₁ is COOH, R₂ is H, and vice versa;
   R₃ and R₅ independently of each other mean H, C₁-C₄-alkyl, C₂-C₃-hydroxyalkyl, CH₂COO⁻, CH₂CH₂CN, CH₂CH₂CONH₂ or CH₂CH₂SO₃⁻;
   R₄ and R₆ independently of each other mean H, C₁-C₄-alkyl, C₂-C₃-hydroxyalkyl, CH₂COO⁻, CH₂CH₂SO₃⁻, CH(COO⁻)CH₂COO⁻, CH(COO⁻)CH₂CH₂COO⁻ or CH₂-phenyl; or
   R₃ and R₄, or R₅ and R₆, independently of each other together with the nitrogen atom form a morpholine ring; and wherein
   M represents the corresponding charge equivalent selected from H⁺, an alkali metal cation, ammonium, C₁-C₄-tetraalkyl ammonium, ammonium which is mono-, di-, tri- or tetra-substituted by C₂-C₃-hydroxyalkyl radicals, and NH(R₇)ₙ(R₈)ₘ⁺, with R₇ being C₁-C₄-alkyl and R₈ being C₂-C₃-hydroxyalkyl, wherein both n and m are an integer of 1 or 2 and n ≠ m;
   as well as mixtures thereof;
   for surface treatment of cellulosic sheet-formed structures.

In preferred embodiments, the use according to the invention comprises the use of the micronized cellulose and the at least one fluorescent whitening agent of formula (1) in aqueous preparations for the production of sized or coated paper or board. The present invention furthermore relates to the combined use of the two substances for the whitening of two-dimensional, cellulosic materials like paper, paperboard and cotton fabric, as well as for the whitening of paper coating colors. Further, the invention relates to preparations, in particular size press liquors, coating colors and coating slips, respectively, containing the micronized cellulose and the at least one fluorescent whitening agent of formula (1). In addition, the invention refers to a process for surface treatment of cellulosic sheet-formed structures, in particular paper or board, and paper or board obtainable by that process.

Preferred embodiments of the invention are described in the description hereinafter, the examples and the claims.

The term micronized cellulose, as used in the present invention, means a cellulose material, wherein the medium fiber length and medium particle size, respectively, is below or equal to 15 µm. The micronized cellulose is essentially free from fibers exceeding a length of 100 µm, and the maximum fiber diameter and maximum particle diameter, respectively, does not exceed 25 µm. Several names and synonyms are used for micronized cellulose, e.g. ultrafine cellulose, nanocellulose, nanofibrillated cellulose (NFC), cellulose nanofiber, nanoscale fibrillated cellulose, microfibrillated cellulose (MFC), and cellulose microfibrils.

Micronized cellulose is commercially available on the market. It can be produced from cellulose fibers by a process involving a step of mechanical disintegration. The starting material can be, for example, chemical pulp or mechanical pulp as it is typically used in the paper industry. The dimensions of cellulose fibers can be characterized by their length and their diameter. Typically, the medium fiber diameter of the starting material is in the range of 15-45 µm and the medium fiber length exceeds 500 µm. Micronized cellulose can generally be produced by carrying out at least one step of mechanical disintegration, optionally combined with a chemical or enzymatic treatment, of the starting material of cellulose fibers. Suitable equipment for this mechanical disintegration are, for example, refiners, grinders, friction grinders, colloid mills, ultrasonic generators, homogenizers, microfluidizers, macrofluidizers and fluidizer-type homogenizers. Any kind of cellulose fibers originating from plants is suitable as starting material, for example cellulose fibers derived from wood, straw, flax or cotton, wherein wood is preferred. The cellulose fibers may be produced from softwood or hardwood. They can be produced according to known methods of cellulose pulp production in form of mechanical, semi-chemical or chemical pulp. It is also possible to use recycled cellulose fibers from recovered paper. The cellulose fibers in the starting material may be present in bleached or unbleached form. Moreover, these fibers may have been treated, at least partially, by a beating or refining process, prior to the mechanical disintegration step to produce the micronized cellulose. It is preferred to use bleached cellulose pulp, in which the lignin deriving from wood has been essentially removed during the pulping process. Lignin has a strong tendency to show yellowing and also can act as UV absorber. Therefore, the combination of lignin-containing micronized cellulose and fluorescent whitening agent is less suitable, but can be used. In a preferred embodiment, the micronized cellulose comprises essentially no lignin. The micronized cellulose may contain a fraction of hemicellulose, originating from the plant material.

Micronized cellulose shows a distinctly different behavior compared to common cellulose pulp. Aqueous dispersions of micronized cellulose are present as viscous, gel-like formulations, wherein the water retention is higher than in common cellulose pulp.

The micronized cellulose used in the present invention has a medium fiber length and medium particle size, respectively, below or equal to 15 µm, preferably in a range of from 0.005 µm to 15 µm. Indications of size, length or diameter for the micronized cellulose can be determined e.g. by scanning electron microscopy (SEM) or by transmission electron microscopy (TEM). In a preferable embodiment, the medium fiber length, respectively medium particle size, is below or equal to 12 µm, in particular in a range of from 0.005 µm to 12 µm. In a particularly preferable embodiment, the medium fiber length, respectively medium particle size, is below or equal to 10 µm, in particular in a range of from 0.005 µm to 10 µm. The micronized cellulose is essentially free, preferably free, from fibers with a length exceeding 100 µm. In a preferred embodiment, the micronized cellulose is essentially free, preferably free, from fibers with a length exceeding 80 µm. The maximum fiber diameter and maximum particle diameter, respectively, does not exceed 25 µm, preferably does not exceed 20 µm.

The fluorescent whitening agents (FWAs) used according to the present invention are those of formula (1). Particularly suitable fluorescent whitening agents of formula (1) are those, wherein R₃, R₄, R₅ and R₆, independently from each other, mean H, methyl, ethyl, n-propyl, iso-propyl, 2-hydroxyethyl, 2-hydroxypropyl, CH₂COO⁻ or CH₂CH₂SO₃⁻, M represents the corresponding charge equivalent, in particular selected from H⁺, Li⁺, Na⁺, K⁺, mono-, di- or triethanolammonium, and R₁ and R₂ are as defined above; or mixtures thereof. In a preferred embodiment, in formula (1) one group of R₁ and R₂ is H and the other group is selected from SO₃⁻ and COOH.

Particularly preferred fluorescent whitening agents have the structure of one of the following formulae (2) to (8): 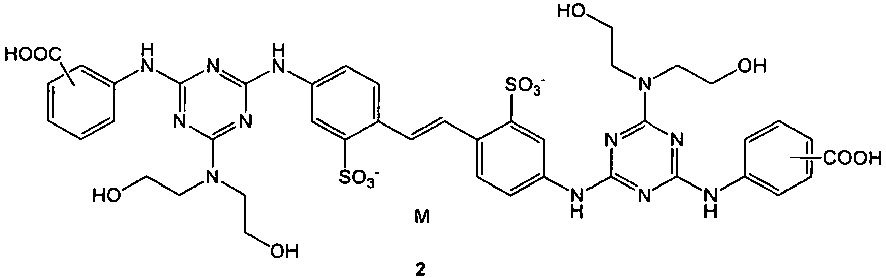 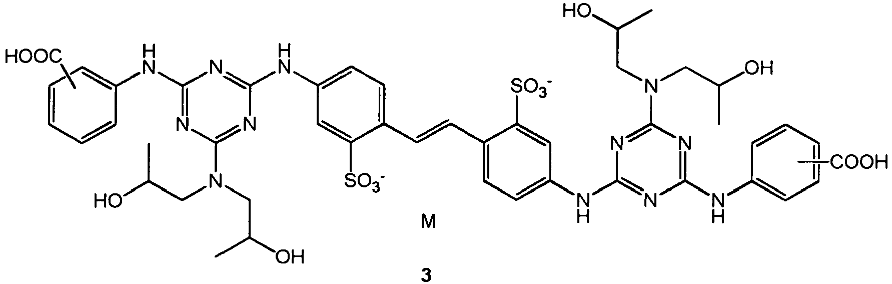 wherein for both formulae (2) and (3), the carboxy groups are, independently from each other, preferably located in *o*- orp-position; 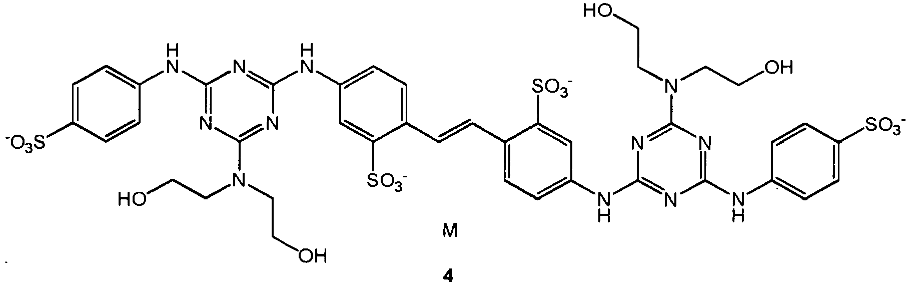 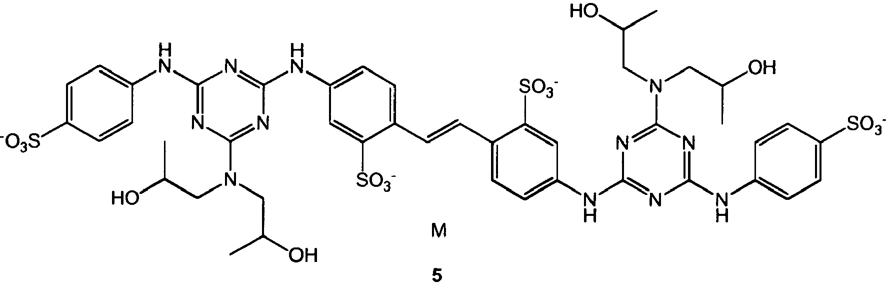 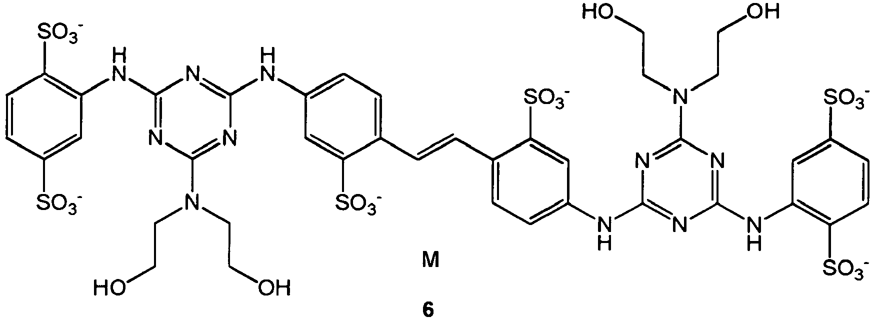 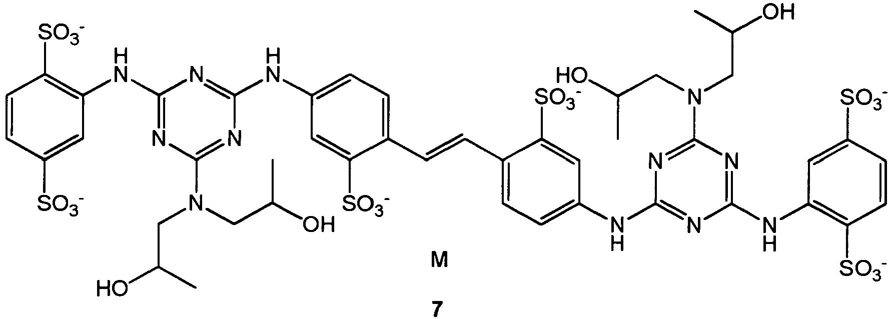 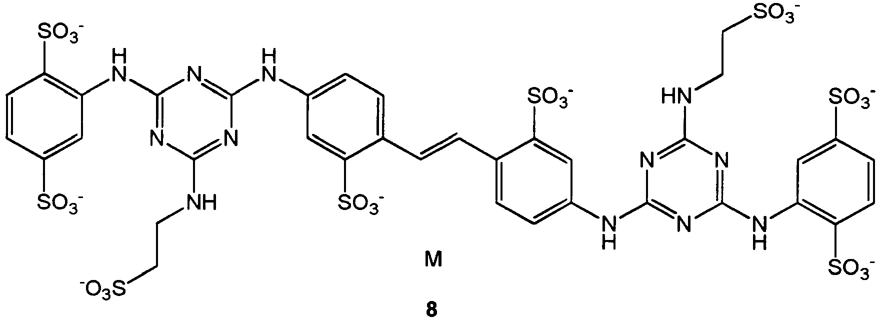 wherein M in each case represents the corresponding charge equivalent, preferably selected from H⁺, Li⁺, Na⁺, K⁺, mono-, di- or triethanolammonium, or mixtures thereof.

The fluorescent whitening agents are preferably used in form of aqueous solutions, aqueous slurries, or dry powders. When starting from a slurry or powder form, preferably the fluorescent whitening agents are dissolved in water prior to contact with the micronized cellulose, to promote an even distribution within the micronized cellulose. The content of the fluorescent whitening agents in the aqueous solutions is preferably below 30 weight-%, in particular from 5 to 25 weight-%. The pH value is preferably in the range from 6 to 10.

The production of fluorescent whitening agents of formula (1) is known in the art. The fluorescent whitening agents can be synthesized according to known procedures in subsequent steps starting from about 1 mol of 4,4'-diamino-2,2'-stilbenedisulfonate disodium salt, about 2 mol cyanuric chloride, and in total about 4 to 4.5 mol of amine compounds corresponding to the amino residues that are linked to the triazine rings in formula (1). The reaction solutions obtained by the synthetic processes contain the fluorescent whitening agents in most cases in form of their sodium salt. The solutions can be furthermore worked up, e.g. by a membrane filtration process, wherein the solution is concentrated and inorganic salts are removed to a large extent. The work-up steps can also involve precipitation or crystallization of the fluorescent whitening agents from the aqueous solution by means of acid addition or salt addition, and subsequent isolation of the fluorescent whitening agents by filtration. The obtained filter cake, commonly after washing, may be formulated as slurry or as aqueous solution of the corresponding fluorescent whitening agent, optionally with addition of bases, solubilizing additives and/or carrier polymers. Those additives may also be added to the fluorescent whitening agent concentrates obtained by membrane filtration. Another option is spray drying of aqueous fluorescent whitening agent solutions in order to produce dry powders.

Generally, preparations of fluorescent whitening agents may contain organic byproducts and inorganic salts arising from the synthesis. Further, additives or auxiliaries may be added, such as carrier polymers, solubilizing additives to improve solubility and long-term storage stability, or biocides to protect the preparations from the growth of bacteria and fungi. Common solubilizing additives are urea, alcohols and amines, for example diethylene glycol, triethylene glycol, propanediol, glycerol, ε-caprolactam, monoethanolamine, diethanolamine or triethanolamine.

According to the present invention it is preferred to use aqueous preparations of the fluorescent whitening agents that are essentially free from solubilizing additives.

In a preferred embodiment, the micronized cellulose is brought into the form of an aqueous dispersion prior to mixing with the fluorescent whitening agents. The amount of micronized cellulose in the aqueous dispersion is preferably in the range from 1 to 30 weight-%, more preferably 3 to 25 weight-%. This aqueous dispersion is then blended with an aqueous fluorescent whitening agent preparation as described above, and agitated to ensure good mixing, to obtain an aqueous preparation or mixture. The mixing time is preferably between 1 and 60 minutes, more preferably between 1 and 20 minutes. The temperature during mixing is preferably in the range of from 10 to 95 °C, more preferably of from 15 to 75 °C. The weight ratio of fluorescent whitening agent to micronized cellulose is preferably in the range of from 0.5 : 99.5 to 80 : 20 weight-%, more preferably from 1 : 99 to 50 : 50 weight-%, in particular from 7 : 93 to 50 : 50 weight-%, more preferred from 10 : 90 to 50 : 50 weight-%, based on the active substance content of the aqueous preparation or mixture.

The mixture or preparation of micronized cellulose in form of its aqueous dispersion and the fluorescent whitening agent in form of its aqueous solution can also be generated directly in the application, in particular in the size press or film press liquor as well as in the coating color during or after its preparation. In case of size press application, the two components of the mixture are added in any sequence or simultaneously to the size press or film press liquor, for example to the working tank. In case of coating application, the micronized cellulose does not need to be dispersed in water prior to its addition, if it can be added to the coating color preparation line at an addition point where thorough mixing occurs. Such thorough agitation is common during the preparation of coating colors, and thus results automatically in the dispersion and homogeneous distribution of the micronized cellulose during the preparation process. The fluorescent whitening agent is preferably added as aqueous solution; prior to, simultaneously to, or after the addition of micronized cellulose.

Depending on the intended use, optionally carrier polymers are added to the mixtures or preparations of micronized cellulose and fluorescent whitening agents. Suitable carrier polymers are linear polyethylene glycols, in particular linear polyethylene glycols with a medium molecular weight Mₙ of 200 to 8000 g/mol, polyvinyl alcohol, carboxymethyl cellulose, polyvinylpyrrolidone, or combinations thereof, wherein linear polyethylene glycols are preferred. In order to improve the long-term storage stability of these mixtures, further additives e.g. thickeners, solubilizers and/or biocides may be added. Furthermore, the pH of these mixtures may be adjusted to the desired value by adding common acids or bases. Suitable pH values are in the range of from 6 to 10.

The cellulosic sheet-formed structure or material that is surface-treated according to the invention can be any material that comprises cellulose fibers and optionally mineral or precipitated fillers, and is formed into a sheet or web, or is two-dimensionally formed. The structure or material can be mainly composed of cellulose fibers, or consist of cellulose fibers. For example, paper and board in form of sheets or cotton textiles in form of webs may be used. In particular, paper stock or paper pulp is not comprised.

A large variety of papers can be used according to the present invention: paper made from unbleached or bleached chemical, semi-chemical or mechanical pulp, e.g. chemical short fiber and long fiber pulp, groundwood, thermo-mechanical pulp (TMP) and chemi-thermo-mechanical pulp (CTMP). Also paper made from unbleached or bleached deinked pulp (DIP) is suitable. DIP is produced from recovered paper. In papermaking, often different grades of mechanical, semi-chemical and chemical pulp as well as recycled pulp are combined in order to impart certain properties to the paper. In addition, the use of inorganic fillers is common in papermaking, on the one hand to save costs for the paper raw materials, on the other hand to improve important functional properties of the paper, like basic brightness, opacity and printability. In the present invention all papers filled with commonly used fillers are suitable, e.g. papers filled with GCC, (ground calcium carbonate), kaolin, PCC (precipitated calcium carbonate), titanium dioxide, talc, barium sulfate, calcium sulfate and satin white.

In case of uncoated paper, the present invention is preferably used for the whitening of printing and writing papers. In case of coated paper and board, generally coated papers of all grades are suitable. In case of cellulosic textiles, the present invention is preferably used for cotton fabric.

The invention relates to the use of the micronized cellulose and the fluorescent whitening agents in a preparation, in particular an aqueous preparation. Further, the present invention relates to such preparations.

The preparations according to the invention preferably comprise the fluorescent whitening agent and micronized cellulose in such amounts, based on weight, that the amount of micronized cellulose is less than fifteen-fold the amount of the fluorescent whitening agent, preferably less than or equal to the tenfold amount of the fluorescent whitening agent, in particular less than or equal to the fivefold amount of the fluorescent whitening agent, in each case based on the active substance content. In another preferred embodiment, the weight ratio of fluorescent whitening agent to micronized cellulose is in the range of from 0.5 : 99.5 to 80 : 20 weight-%, more preferably from 1 : 99 to 50 : 50 weight-%, in particular from 7 : 93 to 50 : 50 weight-%, more preferred from 10 : 90 to 50 : 50 weight-%, based on the active substance content of the aqueous preparation or mixture.

In a preferred embodiment, the preparation according to the invention or the preparation used according to the invention is a size press liquor or film press liquor. These surface treatment liquors are preferably based on starch solutions, wherein the starch concentration ranges up to about 15 weight-%, and in particular is in a range of from 4 to 13 weight-%, based on 100 weight-% of the liquor. The starch used can be any native or degraded starch, which is optionally derivatized. Suitable starches are any starches derived from potatoes, wheat, maize, tapioca, rice, and mixtures thereof. In the surface sizing process, it is preferred to use degraded or derivatized starches. A preferred starch is e.g. dextrin which is thermally degraded starch having a relatively low molecular weight. When using dextrin, it is possible to use higher concentrations of the starch in the liquor, e.g. up to about 30 weight-%. Suitable starch concentrations in the liquors can be adjusted by the skilled person depending on the particular case, e.g. the desired viscosity of the starch solution and/or the desired starch penetration into the paper. Generally, starch degradation can take place by oxidative treatment, e.g. with hypochlorite, by enzymatic, thermal or chemical treatment. Suitable starch derivatives for the surface sizing process are e.g. cationized starches, cationized starches that have been additionally degraded by oxidation, and hydroxyalkyl-substituted starches.

According to the invention the size press or film press liquors contain preferably micronized cellulose in an amount in the range of from 0.01 to 10 weight-%, more preferably from 0.05 to 7.5 weight-%, and most preferably from 0.2 to 5 weight-%, and fluorescent whitening agents preferably in an amount in the range of from 0.02 to 1.4 weight-%, more preferably from 0.03 to 1.2 weight-%, and most preferably from 0.04 and 1.0 weight-%, based on the total amount of size press liquor.

Furthermore, the liquors may contain inorganic salts, in order to adjust the conductivity of the paper, and/or to influence the printing properties, in particular for inkjet printing. Examples for suitable inorganic salts are sodium chloride, sodium sulfate, calcium chloride, magnesium chloride, calcium formate, magnesium formate, and combinations thereof The surface treatment of uncoated paper with water-soluble calcium or magnesium salts improves color fixation during inkjet printing and enables quick uptake of the liquid from the inkjet ink. This in turn leads to an improved inkjet print image regarding color density, brilliancy and edge sharpness.

In addition, according to the invention in particular in case of surface treatment of uncoated paper intended for inkjet printing, salts of bivalent alkaline earth metals may be present in the surface treatment liquors. Suitable salts are described in EP 2 135 997 A1. The alkaline earth metal of the salts is in particular selected from calcium and magnesium. Preferably, the counterions of the bivalent cations are mono- or multivalent anions, in particular halide, sulfate, hydrosulfate, phosphate, hydrophosphate, dihydrophosphate, carbonate, hydrocarbonate, nitrate, formate, acetate, or a mixture thereof, preferably chloride or sulfate, most preferably chloride. The salts disclosed in US 6,207,258 B1 are also suitable. A preferred salt is calcium chloride, magnesium chloride, magnesium sulfate, or a mixture thereof; more preferred is calcium chloride, magnesium chloride, or a mixture thereof; most preferred is calcium chloride. When such salts are added, they are added in dissolved form, usually in aqueous solution, when preparing the surface treatment liquor. The salts serve to obtain an improved print image during inkjet printing on the treated paper, especially with regard to color density, brilliancy and edge sharpness.

Furthermore, the surface treatment liquors may contain defoamers and synthetic sizing agents. These sizing agents can be applied to adjust the paper's uptake of water-based liquids during the writing and printing process in a controlled manner. Commonly used paper surface sizing agents are polymer dispersions and polymer solutions containing polymers of the styrene-acrylic ester type, the styrene-acrylic acid type and the styrene-maleic anhydride type. Further, as a common sizing agent alkylketene dimer (AKD) may be used, either as such or in combination with the above mentioned sizing polymers. Optionally, the treatment liquors may contain additives to impart special functional properties to the treated paper, like barrier effects and oleophobic behaviour. Such additives can be fluorocarbons, waxes or other paper additives that lead to barrier properties. In some cases, carrier polymers are added to the surface treatment liquors, for example linear polyethylene glycols or polyvinyl alcohol. Preferably, sizing agents are present in amounts of about 0.1 to 3 weight-% in the size press liquor, relating to the sizing agent solution or dispersion. Carrier polymers, if used, are present in amounts of about 0.2 to 5 weight-% in the size press liquor.

The surface treatment, respectively surface sizing, of uncoated paper is usually carried out with an application device that is part of the paper machine. Suitable surface treatment devices are all common devices that are used in the paper industry for surface sizing, for example the size press and the film press.

In a further preferred embodiment, the preparation according to the invention or the preparation used according to the invention is a coating color. Suitable coating colors are all common paper coating colors, based on white pigments and binders. Examples for suitable white pigments to produce coating colors are GCC, kaolin respectively clay, calcined clay, PCC, talc, titanium dioxide and calcium sulfate. Examples for suitable binders are, on one hand, polymer dispersions, respectively latices, based on polymer types selected from styrene-butadiene, styrene-acrylic ester, vinyl acetate and vinyl acetate-acrylic ester. On the other hand, modified starches and dextrins, as described above in the context of the size press liquor, may be used as binders, optionally in combination with the above mentioned polymer dispersions. In some cases, casein may be used as binder. Coating colors furthermore may contain co-binders, e.g. polyvinyl alcohol and/or carboxymethyl cellulose.

In addition to the white pigments and binders, the coating colors preferably contain dispersing agents. Dispersing agents are added to stabilize the pigment particles. Suitable dispersing agents are polyacrylic acid or modified polyacrylic acid in form of their salts, oligophosphates and polyphosphates. The coating color can be prepared, starting from the white pigments in dry form, by dispersing the white pigments in water to form the corresponding pigment slurries. Thereafter, the components of the coating color are blended. Often the major part of dispersing agent is already contained in the pigment slurry that is used as starting material to prepare the coating color.

The coating colors may contain further additives or auxiliaries. The coating colors may contain thickening substances, in order to adjust a certain desired viscosity needed for further processing, and also a certain degree of water retention. Examples for suitable thickeners are carboxymethyl cellulose, alginates, or fully synthetic thickener polymers based on acrylates. Further common coating color additives are tinting dyes or tinting pigments, defoamers and optionally stearates.

According to the invention the coating colors contain micronized cellulose preferably in an amount in the range of from 0.01 to 8 weight-%, more preferably from 0.02 to 6 weight-%, and most preferably from 0.04 to 4 weight-%, and fluorescent whitening agents preferably in an amount in the range of from 0.02 to 1 weight-%, more preferably from 0.03 and 0.875 weight-%, and most preferably from 0.04 and 0.75 weight-%, based on pigment content of the coating color.

In the present invention for the surface treatment all paper coating processes commonly used or known in the art are suitable, for example film press coating, blade coating, curtain coating and spray coating.

In addition to size press liquors and coating colors and the related surface treatments, the invention comprises treatment liquors suitable for pigmentation. The term pigmentation describes a process common in the art, wherein paper surface is treated in a similar way as in the above described surface sizing process, but with the difference that pigments are an essential part of the treatment liquor. That pigmentation liquor preferably contains one or more binders, selected from starch including its derivatized or degraded forms, and polymer dispersions based on polymer types selected from styrene-butadiene, styrene-acrylic ester, vinyl acetate and vinyl acetate-acrylic ester. The term paper coating is commonly used when coating colors form a fully closed layer on the base paper having a coat weight above approx. 5-6 g/m². The coat weight during the pigmentation process is commonly below 5-6 g/m². The pigmentation of a base paper is typically used to improve its optical properties, as well as the printability and the evenness of the paper surface.

In case of the treatment of cellulosic textiles, all common and known methods that are typically used for the surface treatment of those textiles with fluorescent whitening agents are suitable in the context of the present invention. Suitable methods are the continuous process, the semi-continuous process, the exhaustion process and the foulard treatment, wherein the whitening may be combined with e.g. peroxide bleaching, with a starch finish or a high temperature process.

The invention is illustrated by the following examples which describe preferred embodiments without restricting the scope of the invention. All percentages are indicated in weight-%, unless otherwise indicated.

### Examples

### Example 1

This example illustrates the use of micronized cellulose and fluorescent whitening agent in size press application.

First, an aqueous dispersion of the micronized cellulose Arbocel^{®} UFC 100 (supplied by J. Rettenmaier & Söhne; cellulose content approx. 99.5%; medium particle size approx. 8 µm) was produced. 15 g of Arbocel^{®} UFC 100 were stirred into 85 g of demineralized water, followed by dispersing with an Ultra-Turrax device at 20000 rpm for 30 seconds. A dispersion having a solids content of 14.9% was obtained. An aqueous fluorescent whitening agent (FWA) preparation containing the fluorescent whitening agent of formula (4), as shown above, in form of its sodium salt as active ingredient and having a fluorescent whitening agent content of about 21% was used for the following Mixtures 1a-1d.

For this, 67.0 g of the above described dispersion of micronized cellulose were used for each mixture, blended with the amounts of the aqueous fluorescent whitening agent (FWA) preparation as mentioned below, and then filled up to 100.0 g using demineralized water in each case.
Mixture 1a: 7.5 g FWA preparation
Mixture 1b: 15.0 g FWA preparation
Mixture 1c: 22.5 g FWA preparation
Mixture 1d: 30.0 g FWA preparation

The mixtures were then stirred in each case for 5 minutes at room temperature by using a magnetic stirrer.

Size press liquors were produced as follows: 25.0 g of each of the above mentioned Mixtures 1a-1d were blended at room temperature with 192.3 g of an aqueous solution of the starch Perfectamyl^{®} A 4692 (from AVEBE) having a concentration of 6.5%, followed by filling up to 250.0 g using demineralized water, and finally stirring for 5 minutes using a magnetic stirrer.

Wood-free coating base paper having a basis weight of 85 g/m² was used for the surface sizing trials. The size press liquors were applied with a laboratory size press (from the company Mathis, type HF) using a speed of 2 m/min and a pressure of 2.5 bar. By weighing the paper directly before and after the size press treatment, a wet pickup of the paper of approx. 40% for all four treatment liquors was calculated.

The treated papers were dried in each case on a drying cylinder for 2 minutes at 105 °C, and afterwards stored in standard climate (23 °C, 50 % relative humidity) for 24 hours. Then, ISO brightness and CIE whiteness values were determined using a Datacolor ELREPHO 2000 device.

In an analogous manner, an aqueous fluorescent whitening agent (FWA) preparation of the fluorescent whitening agent of formula (6), as shown above, in form of its sodium salt and having a fluorescent whitening agent content of approx. 15%, was used to produce the Mixtures 1e-1h. In each case, the amounts of FWA preparation as mentioned below were blended with 20.1 g of the dispersion of micronized cellulose.
Mixture 1e: 5.95 g FWA preparation
Mixture 1f: 11.9 g FWA preparation
Mixture 1g: 17.9 g FWA preparation
Mixture 1h: 23.9 g FWA preparation

The mixtures were in each case filled up to 100.0 g using demineralized water, followed by stirring for 10 minutes and following the above described procedure.

For this, 50.0 g of each mixture were blended with 192.3 g of the solution of Perfectamyl^{®} A 4692 (concentration 6.5%), filled up to 250.0 g using demineralized water and stirred as described above.

The surface sizing process was carried out in a similar way as described above, the only difference being that each paper sheet was passed through the size press for two times subsequently. This led to a wet pickup of the paper of approx. 67% for each of the four treatment liquors.

The results obtained are shown in Table 1 below.

### Example 2

This example illustrates the use of micronized cellulose and fluorescent whitening agent in size press application, wherein the mixing of both components was carried out during preparation of the size press liquor.

The same fluorescent whitening agent (FWA) preparations and the same aqueous dispersion of micronized cellulose as described in Example 1 above were used.

The weight ratio between the FWA preparations (containing the FWA of formula (4) and formula (6), respectively) and micronized cellulose was for each case identical to that of Example 1. The respective mixing of both components was, however, carried out directly during the preparation of the size press liquor. For this purpose, both respective components were added shortly after each other to an aqueous solution of Perfectamyl^{®} A 4692, having a concentration of 6.5%.

### Mixtures 2a-2d: the aqueous FWA preparation of the FWA having formula (4) was used

### Mixtures 2e-2h: the aqueous FWA preparation of the FWA having formula (6) was used

In each case, 192.3 g of starch solution were first placed in a glass beaker, and then blended with the following amounts of FWA preparation and dispersion of micronized cellulose:
Mixture 2a: 1.875 g FWA preparation + 16.8 g dispersion of micronized cellulose
Mixture 2b: 3.75 g FWA preparation + 16.8 g dispersion of micronized cellulose
Mixture 2c: 5.625 g FWA preparation + 16.8 g dispersion of micronized cellulose
Mixture 2d: 7.50 g FWA preparation + 16.8 g dispersion of micronized cellulose
Mixture 2e: 2.99 g FWA preparation + 10.1 g dispersion of micronized cellulose
Mixture 2f: 5.97 g FWA preparation + 10.1 g dispersion of micronized cellulose
Mixture 2g: 8.95 g FWA preparation + 10.1 g dispersion of micronized cellulose
Mixture 2h: 11.94 g FWA preparation + 10.1 g dispersion of micronized cellulose

The preparation of the size press liquors were carried out at room temperature. The mixing was carried out using a magnetic stirrer. The dispersion of micronized cellulose was added in each case first to the starch solution under stirring. After approx. 1 minute of mixing time, the corresponding FWA preparation in the corresponding amount was added. In each case, the batches were then filled up to 250.0 g using demineralized water and stirred for another 15 minutes.

The paper surface sizing, drying and whiteness evaluation were carried out analogously to Example 1, wherein the papers were passed once through the size press in case of the Mixtures 2a-2d (wet pickup approx. 40%), and twice in case of the Mixtures 2e-2h (wet pickup approx. 67%).

The results obtained are shown in Table I below.

### Comparative Example 1

The same FWA preparations and the same starch solution as described in Examples 1 and 2 were used.

The FWA amounts used in the size press liquors were identical to that of Example 2. However, no micronized cellulose was used. Both aqueous FWA preparations were added in the amounts as mentioned in Example 2 at room temperature to 192.3 g of starch solution in each case. Then, all batches were filled up to 250.0 g using demineralized water and stirred using a magnetic stirrer for another 10 minutes.

### Mixtures C1a-C1d: the aqueous FWA preparation of the FWA having formula (4) was used

### Mixtures C1e-C1h: the aqueous FWA preparation of the FWA having formula (6) was used

The paper surface sizing, drying and whiteness evaluation were carried out analogously to Examples 1 and 2, wherein the papers were passed once through the size press in case of Mixtures C1a-C1d (wet pickup approx. 40%), and twice in case of Examples Mixtures C1e-C1h (wet pickup approx. 67%).

ISO brightness and CIE whiteness values of the mixtures of Examples 1, 2 and Comparative Example 1 are shown in the following Table 1.

**Table 1**

| Used mixture according to example | FWA structure | FWA amount in the size press liquor (%) | Amount of micronized cellulose in the size press liquor (%) | ISO Brightness (%) | CIE Whiteness |
|---|---|---|---|---|---|
| 1a | 4 | 0.158 | 1.0 | 96.0 | 121.8 |
| 1b | 4 | 0.315 | 1.0 | 98.3 | 127.2 |
| 1c | 4 | 0.473 | 1.0 | 99.5 | 128.8 |
| 1d | 4 | 0.630 | 1.0 | 100.2 | 129.7 |
| 2a | 4 | 0.158 | 1.0 | 96.1 | 122.3 |
| 2b | 4 | 0.315 | 1.0 | 98.4 | 126.9 |
| 2c | 4 | 0.473 | 1.0 | 99.6 | 129.0 |
| 2d | 4 | 0.630 | 1.0 | 100.1 | 128.9 |
| C1a | 4 | 0.158 | without | 95.3 | 119.9 |
| C1b | 4 | 0.315 | without | 97.5 | 125.2 |
| C1c | 4 | 0.473 | without | 99.0 | 127.8 |
| C1d | 4 | 0.630 | without | 99.6 | 128.0 |
| 1e | 6 | 0.178 | 0.6 | 98.3 | 128.8 |
| 1f | 6 | 0.357 | 0.6 | 101.3 | 135.5 |
| 1g | 6 | 0.537 | 0.6 | 103.0 | 139.2 |
| 1h | 6 | 0.717 | 0.6 | 103.7 | 140.2 |
| 2e | 6 | 0.179 | 0.6 | 98.5 | 129.2 |
| 2f | 6 | 0.358 | 0.6 | 101.5 | 136.0 |
| 2g | 6 | 0.537 | 0.6 | 103.0 | 139.4 |
| 2h | 6 | 0.716 | 0.6 | 104.0 | 140.8 |
| C1e | 6 | 0.179 | without | 98.0 | 128.3 |
| C1f | 6 | 0.358 | without | 100.9 | 134.8 |
| C1g | 6 | 0.537 | without | 102.6 | 138.1 |
| C1h | 6 | 0.716 | without | 103.4 | 139.7 |

As to be seen, the mixtures of micronized cellulose and fluorescent whitening agent (FWA) lead to improved brightness and whiteness of the paper over a broad range of FWA dosage (Mixtures 1a to 1h, Mixtures 2a to 2h), compared to the prior art application of FWAs using no micronized cellulose (Mixtures C1a to C1h). Furthermore, the addition of both components (FWA, micronized cellulose) to the basic size press liquor (starch solution; Mixtures 2a to 2h) yields comparably good results as the separate production of the mixture of both components before adding to the starch solution (Mixtures 1a to 1h).

### Example 3

This example illustrates the use of micronized cellulose and fluorescent whitening agent in coating colour application.

A paper coating colour was prepared from the following raw materials:
482 g of the GCC powder Hydrocarb^{®} 90-OG (from Omya) 127 g of the kaolin "Kaolin KN 83 Granulat" (kaolin content 99%, from Amberger Kaolinwerke)
3.4 g of the dispersant Polysalz^{®} S (approx. 40% solids, from BASF)
11.6 g of the micronized cellulose Arbocel^{®} UFC 100 (from J. Rettenmaier & Sohne)
96 g of the binder Litex^{®} P 7110 (styrene-butadiene latex with approx. 50% solids, from Synthomer)
60 g of a starch solution with 20% solids, prepared from the starch Perfectamyl^{®} A 4692 (from AVEBE)
235.4 g of demineralized water
2.9 g of aqueous sodium hydroxide solution with a strength of 5%

For preparation of 500 g of a 20% starch solution, the starch was stirred first into demineralized water at room temperature by using the corresponding amounts. Stirring was continued at room temperature until a starch slurry free from lumps was formed. Then the starch slurry was heated up to 95 °C and held at this temperature for 25 minutes. The starch solution was then cooled down to 30 °C. Determination of the dry solids gave a value of 20.9%. In order to adjust a solids value of 20.0%, the corresponding amount of demineralized water was added under stirring.

For preparing the coating colour, the above mentioned amounts were mixed in the following manner:
Polysalz^{®} S was first stirred into demineralized water. To this mixture, GCC and kaolin were added and mixed in with a dissolver plate at 500 rpm for 5 minutes.

Then the mixture was dispersed with an Ultra-Turrax at 7000 rpm for 2 minutes. After this, the binder and the starch solution were added subsequently under stirring. The pH of the coating colour was adjusted to 8.5 by adding the aqueous sodium hydroxide solution. Stirring was continued for another 5 minutes. A paper coating colour with a solids content of 67.0% and a pigment content of 59.7% was obtained.

The coating colour was separated into 5 portions of 200 g. To each portion, an aqueous preparation of the FWA of formula (5), as shown above, in form of its sodium salt was added in the amounts mentioned below and stirred in for 5 minutes. The FWA preparation had an active substance content of about 22.8%.
Coating colour 3a: 0.263 g of FWA preparation
Coating colour 3b: 0.526 g of FWA preparation
Coating colour 3c: 1.051 g of FWA preparation
Coating colour 3d: 2.102 g of FWA preparation
Coating colour 3e: 3.154 g of FWA preparation

The coating colours were each applied to wood-free base paper sheets having a basis weight of approx. 85 g/m². For this purpose, the laboratory coater Erichsen K-Control-Coater, model K 202, was used. The coated papers were then dried on a drum dryer at 95 °C for 1 minute and afterwards stored for 4 hours at 23 °C and a relative humidity of 50%. The applied coat weight was approx. 15 g/m².

Then, CIE whiteness and ISO brightness values were determined by using a Datacolor ELREPHO 2000 device.

The results obtained are shown in Table 2 below.

### Comparative Example 2

A paper coating colour was prepared in analogous manner as described in Example 3, but without using micronized cellulose. The amounts of the coating colour ingredients were the same as mentioned in Example 3, except for the amount of demineralized water and that no micronized cellulose was used. The amount of demineralized water was 247.0 g. A paper coating colour with a solids content of 65.8% and a pigment content of 59.7% was obtained.

The coating colour was separated into 5 portions of 200 g. To each portion, an aqueous preparation of the FWA of formula (5), as shown above, in form of its sodium salt was added in the amounts mentioned below and stirred in for 5 minutes. The FWA preparation had an active substance content of about 22.8%.
Coating colour C2a: 0.263 g of FWA preparation
Coating colour C2b: 0.526 g of FWA preparation
Coating colour C2c: 1.051 g of FWA preparation
Coating colour C2d: 2.102 g of FWA preparation
Coating colour C2e: 3.154 g of FWA preparation

The preparation of the coated papers and the whiteness and brightness measurement were carried out in the same manner as described in Example 3.

The CIE whiteness and ISO brightness values obtained are listed in the following Table 2.

**Table 2**

| Used mixture according to example | FWA structure | FWA amount in the coating colour (% related to dry pigment) | Amount of micronized cellulose in the coating colour (% related to dry pigment) | ISO Brightness (%) | CIE Whiteness |
|---|---|---|---|---|---|
| 3a | 5 | 0.050 | 1.9 | 89.4 | 96.5 |
| 3b | 5 | 0.100 | 1.9 | 90.7 | 100.6 |
| 3c | 5 | 0.200 | 1.9 | 92.7 | 106.5 |
| 3d | 5 | 0.400 | 1.9 | 95.4 | 113.9 |
| 3e | 5 | 0.600 | 1.9 | 96.3 | 115.3 |
| C2a | 5 | 0.050 | without | 88.5 | 93.5 |
| C2b | 5 | 0.100 | without | 89.9 | 98.1 |
| C2c | 5 | 0.200 | without | 92.1 | 104.8 |
| C2d | 5 | 0.400 | without | 94.3 | 110.5 |
| C2e | 5 | 0.600 | without | 95.6 | 113.0 |

It is apparent that the combined use of micronized cellulose and FWA leads to improved brightness and whiteness values of the coated paper over a broad range of FWA amounts.

## Claims

1. Use of micronized cellulose having a medium fiber length and medium particle size, respectively, below or equal to 15 µm, having a maximum fiber diameter and maximum particle diameter, respectively, not exceeding 25 µm and being essentially free from fibers with a length exceeding 100 µm, and at least one fluorescent whitening agent (FWA) having the formula (1) 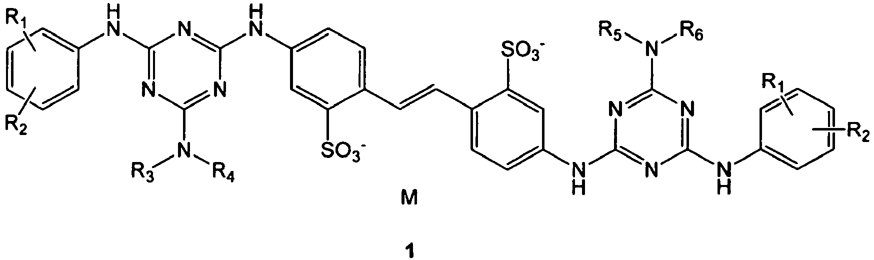 wherein
R₁ and R₂ independently of each other mean H, SO₃⁻ or COOH, with the proviso that, if R₁ is COOH, R₂ is H, and vice versa;
R₃ and R₅ independently of each other mean H, C₁-C₄-alkyl, C₂-C₃-hydroxyalkyl, CH₂COO⁻, CH₂CH₂CN, CH₂CH₂CONH₂ or CH₂CH₂SO₃⁻;
R₄ and R₆ independently of each other mean H, C₁-C₄-alkyl, C₂-C₃-hydroxyalkyl, CH₂COO⁻, CH₂CH₂SO₃⁻, CH(COO⁻)CH₂COO⁻, CH(COO⁻)CH₂CH₂COO⁻ or CH₂-phenyl; or
R₃ and R₄, or R₅ and R₆, independently of each other together with the nitrogen atom form a morpholine ring; and wherein
M represents the corresponding charge equivalent selected from H⁺, an alkali metal cation, ammonium, C₁-C₄-tetraalkyl ammonium, ammonium which is mono-, di-, tri- or tetra-substituted by C₂-C₃-hydroxyalkyl radicals, and NH(R₇)ₙ(Rs)ₘ⁺, with R₇ being C₁-C₄-alkyl and R₈ being C₂-C₃-hydroxyalkyl, wherein both n and m are an integer of 1 or 2 and n ≠ m;
as well as mixtures thereof; for surface treatment of cellulosic sheet-formed structures.

2. Use according to claim 1, wherein the micronized cellulose and the at least one fluorescent whitening agent are used in a preparation, in particular an aqueous preparation.

3. Use according to claim 2, wherein the preparation is a size press liquor or film press liquor.

4. Use according to claim 2, wherein the preparation is a coating color and coating slip, respectively.

5. Use according to any of the preceding claims, wherein for the micronized cellulose the medium fiber length and medium particle size, respectively, is in a range of from 0.005 µm to 15 µm, and preferably the maximum fiber diameter and maximum particle diameter, respectively, does not exceed 20 µm.

6. Use according to any of the preceding claims, wherein in formula (1) of the at least one fluorescent whitening agent one group of R₁ and R₂ is H and the other group is selected from SO₃⁻ and COOH.

7. Use according to any of the preceding claims, wherein the cellulosic sheet-formed structure is paper or board.

8. Use according to any of claims 1, 2, 5 or 6, wherein the cellulosic sheet-formed structure is textile material, in particular cotton fabric.

9. A preparation for surface whitening treatment of sheet-formed cellulose-containing structures, wherein the preparation comprises micronized cellulose having a medium fiber length and medium particle size, respectively, below or equal to 15 µm, having a maximum fiber diameter and maximum particle diameter, respectively, not exceeding 25 µm and being essentially free from fibers with a length exceeding 100 µm, and at least one fluorescent whitening agent (FWA) having the formula (1) 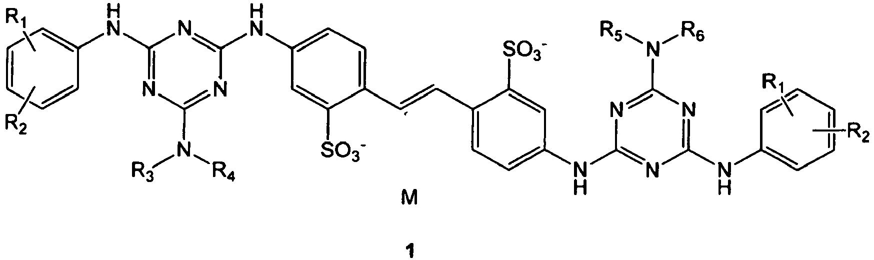 wherein
R₁ and R₂ independently of each other mean H, SO₃⁻ or COOH, with the proviso that, if R₁ is COOH, R₂ is H, and vice versa;
R₃ and R₅ independently of each other mean H, C₁-C₄-alkyl, C₂-C₃-hydroxyalkyl, CH₂COO⁻, CH₂CH₂CN, CH₂CH₂CONH₂ or CH₂CH₂SO₃⁻;
R₄ and R₆ independently of each other mean H, C₁-C₄-alkyl, C₂-C₃-hydroxyalkyl, CH₂COO⁻, CH₂CH₂SO₃⁻, CH(COO⁻)CH₂COO⁻, CH(COO⁻)CH₂CH₂COO⁻ or CH₂-phenyl; or
R₃ and R₄, or R₅ and R₆, independently of each other together with the nitrogen atom form a morpholine ring; and wherein
M represents the corresponding charge equivalent selected from H⁺, an alkali metal cation, ammonium, C₁-C₄-tetraalkyl ammonium, ammonium which is mono-, di-, tri- or tetra-substituted by C₂-C₃-hydroxyalkyl radicals, and NH(R₇)ₙ(R₈)ₘ⁺, with R₇ being C₁-C₄-alkyl and R₈ being C₂-C₃-hydroxyalkyl, wherein both n and m are an integer of 1 or 2 and n ≠ m.

10. The preparation of claim 9, wherein, based on weight, the amount of micronized cellulose is less than or equal to the tenfold amount of the fluorescent whitening agent.

11. The preparation of claim 9, wherein the weight ratio of fluorescent whitening agent to micronized cellulose is in a range of from 7 : 93 to 50 : 50 weight-%, preferably from 10 : 90 to 50 : 50 weight-%, based on the active substance content of the preparation.

12. The preparation of any of claims 9 to 11, wherein the preparation further comprises starch and is a size press liquor or film press liquor.

13. The preparation of any of claims 9 to 11, wherein the preparation further comprises a white pigment and is a coating color and coating slip, respectively.

14. A process for surface whitening treatment of cellulosic sheet-formed structures, comprising treating the cellulosic sheet-formed structure with a preparation as defined in any of claims 9 to 13.

15. A paper obtainable by the process according to claim 14.

## Patentansprüche

1. Verwendung von mikronisierter Cellulose mit einer mittleren Faserlänge bzw. mittleren Teilchengröße unterhalb oder gleich 15 µm, mit einem maximalen Faserdurchmesser bzw. maximalen Teilchendurchmesser von nicht mehr als 25 µm, und welche im Wesentlichen frei von Fasern mit einer Länge von mehr als 100 µm ist, und wenigstens einem fluoreszierenden Weißtöner (FWA) mit der Formel (1) 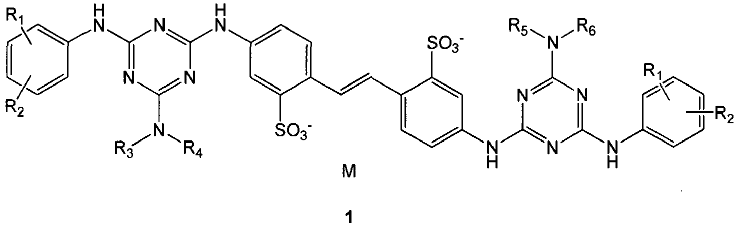 wobei
R₁ und R₂ unabhängig voneinander H, SO₃⁻ oder COOH bedeuten mit der Maßgabe, dass, wenn R₁ COOH ist, R₂ H ist, und umgekehrt;
R₃ und R₅ unabhängig voneinander H, C₁-C₄-Alkyl, C₂-C₃-Hydroxyalkyl, CH₂COO⁻, CH₂CH₂CN, CH₂CH₂CONH₂ oder CH₂CH₂SO₃⁻ bedeuten;
R₄ und R₆ unabhängig voneinander H, C₁-C₄-Alkyl, C₂-C₃-Hydroxyalkyl, CH₂COO⁻, CH₂CH₂SO₃⁻, CH(COO⁻)CH₂COO⁻, CH(COO⁻)CH₂CH₂COO⁻ oder CH₂-Phenyl bedeuten; oder
R₃ und R₄, oder R₅ und R₆, unabhängig voneinander zusammen mit dem Stickstoffatom einen Morpholinring bilden; und wobei
M das entsprechende Ladungsäquivalent darstellt, ausgewählt aus H⁺, einem Alkalimetallkation, Ammonium, C₁-C₄-Tetraalkylammonium, Ammonium, welches mit C₂-C₃-Hydroxyalkyl-Radikalen mono-, di-, tri- oder tetra-substituiert ist, und NH(R₇)ₙ(R₈)ₘ⁺, wobei R₇ C₁-C₄-Alkyl ist und R₈ C₂-C₃-Hydroxyalkyl ist, wobei sowohl n als auch m eine ganze Zahl von 1 oder 2 sind und n ≠ m;
sowie Gemische davon;
zur Oberflächenbehandlung von cellulosischen flächigen bzw. blattgebildeten Strukturen.

2. Verwendung nach Anspruch 1, wobei die mikronisierte Cellulose und der wenigstens eine fluoreszierende Weißtöner in einer Präparation verwendet werden, insbesondere einer wässrigen Präparation.

3. Verwendung nach Anspruch 2, wobei die Präparation eine Leimpressenflotte oder Filmpressenflotte ist.

4. Verwendung nach Anspruch 2, wobei die Präparation eine Streichfarbe bzw. eine Streichmasse ist.

5. Verwendung nach irgendeinem der vorhergehenden Ansprüche, wobei für die mikronisierte Cellulose die mittlere Faserlänge bzw. mittlere Teilchengröße in einem Bereich von 0,005 µum bis 15 µm liegt und vorzugsweise der maximale Faserdurchmesser bzw. maximale Teilchendurchmesser nicht 20 µm übersteigt.

6. Verwendung nach irgendeinem der vorhergehenden Ansprüche, wobei in Formel (1) von dem wenigstens einen fluoreszierenden Weißtöner eine Gruppe von R₁ und R₂ H ist und die andere Gruppe aus SO₃⁻ und COOH ausgewählt ist.

7. Verwendung nach irgendeinem der vorhergehenden Ansprüche, wobei die cellulosische flächige Struktur Papier oder Pappe ist.

8. Verwendung nach irgendeinem der Ansprüche 1, 2, 5 oder 6, wobei die cellulosische flächige Struktur ein Textilmaterial ist, insbesondere Baumwollgewebe.

9. Präparation zur Oberflächenweißtönungsbehandlung von flächigen bzw. blattgebildeten Cellulose-haltigen Strukturen, wobei die Präparation mikronisierte Cellulose mit einer mittleren Faserlänge bzw. mittleren Teilchengröße unterhalb oder gleich 15 µm, mit einem maximalen Faserdurchmesser bzw. maximalen Teilchendurchmesser von nicht mehr als 25 µm, und welche im Wesentlichen frei von Fasern mit einer Länge von mehr als 100 µm ist, und wenigstens einen fluoreszierenden Weißtöner (FWA) mit der Formel (1) umfasst 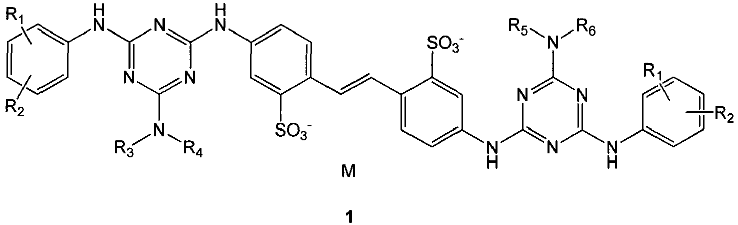 wobei
R₁ und R₂ unabhängig voneinander H, SO₃⁻ oder COOH bedeuten mit der Maßgabe, dass, wenn R₁ COOH ist, R₂ H ist, und umgekehrt;
R₃ und R₅ unabhängig voneinander H, C₁-C₄-Alkyl, C₂-C₃-Hydroxyalkyl, CH₂COO⁻, CH₂CH₂CN, CH₂CH₂CONH₂ oder CH₂CH₂SO₃⁻ bedeuten;
R₄ und R₆ unabhängig voneinander H, C₁-C₄-Alkyl, C₂-C₃-Hydroxyalkyl, CH₂COO⁻, CH₂CH₂SO₃⁻, CH(COO⁻)CH₂COO⁻, CH(COO⁻)CH₂CH₂COO⁻ oder CH₂-Phenyl bedeuten; oder
R₃ und R₄, oder R₅ und R₆, unabhängig voneinander zusammen mit dem Stickstoffatom einen Morpholinring bilden; und wobei
M das entsprechende Ladungsäquivalent darstellt, ausgewählt aus H⁺, einem Alkalimetallkation, Ammonium, C₁-C₄-Tetraalkylammonium, Ammonium, welches mit C₂-C₃-Hydroxyalkyl-Radikalen mono-, di-, tri- oder tetra-substituiert ist, und NH(R₇)ₙ(R₈)ₘ⁺, wobei R₇ C₁-C₄-Alkyl ist und R₈ C₂-C₃-Hydroxyalkyl ist, wobei sowohl n als auch m eine ganze Zahl von 1 oder 2 sind und n * m.

10. Präparation nach Anspruch 9, wobei, bezogen auf das Gewicht, die Menge der mikronisierten Cellulose weniger als die zehnfache oder gleich der zehnfachen Menge des fluoreszierenden Weißtöners ist.

11. Präparation nach Anspruch 9, wobei das Gewichtsverhältnis von fluoreszierendem Weißtöner zu mikronisierter Cellulose in einem Bereich vorn 7 : 93 bis 50 : 50 Gewichts-% liegt, vorzugsweise von 10 : 90 bis 50 : 50 Gewichts-%, bezogen auf Aktivsubstanzgehalt der Präparation.

12. Präparation nach irgendeinem der Ansprüche 9 bis 11, wobei die Präparation weiterhin Stärke umfasst und eine Leimpressenflotte oder Filmpressenflotte ist.

13. Präparation nach irgendeinem der Ansprüche 9 bis 11, wobei die Präparation weiterhin ein Weißpigment umfasst und eine Streichfarbe bzw. Streichmasse ist.

14. Verfahren zur Oberflächenweißtönungsbehandlung von cellulosischen flächigen bzw. blattgebildeten Strukturen, umfassend Behandeln der cellulosischen flächigen Struktur mit einer Präparation wie in irgendeinem der Ansprüche 9 bis 13 definiert.

15. Papier, erhältlich durch das Verfahren gemäß Anspruch 14.

## Revendications

1. Utilisation de cellulose micronisée ayant une longueur moyenne de fibre et une granulométrie moyenne respectivement inférieures ou égales à 15 µm, ayant un diamètre maximal de fibre et une granulométrie maximale ne dépassant pas respectivement 25 µm, et étant essentiellement exempte de fibres ayant une longueur dépassant 100 µm, et d'au moins un agent azurant fluorescent (FWA) ayant la formule (1) 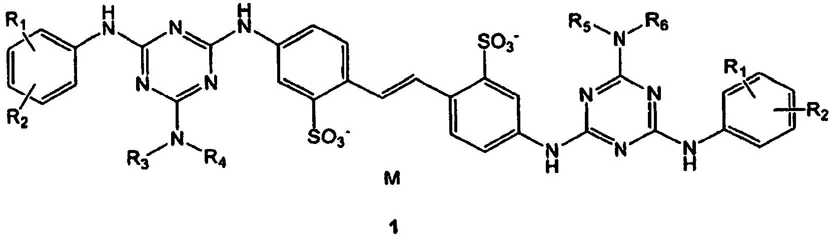 dans laquelle
R₁ et R₂ représentent chacun indépendamment de l'autre H, SO₃⁻ ou COOH, à la condition que, si R₁ est COOH, R₂ soit H, et inversement ;
R₃ et R₅ représentent chacun indépendamment de l'autre H, un groupe alkyle en C₁-C₄, hydroxyalkyle en C₂-C₃, CH₂COO⁻, CH₂CH₂CN, CH₂CH₂CONH₂ ou CH₂CH₂SO₃⁻ ;
R₄ et R₆ représentent chacun indépendamment de l'autre H, un groupe alkyle en C₁-C₄, hydroxyalkyle en C₂-C₃, CH₂COO⁻, CH₂CH₂SO₃⁻, CH(COO⁻)CH₂COO⁻, CH(COO⁻)CH₂CH₂COO⁻, ou CH₂-phényle ; ou
R₃ et R₄, ou R₅ et R₆, indépendamment les uns des autres, forment ensemble avec l'atome d'azote un noyau morpholine ; et dans laquelle
M représente l'équivalent de charge correspondant choisi parmi H⁺, un cation de métal alcalin, ammonium, tétra(alkyle en C₁-C₄) ammonium, ammonium mono-, di-, triou tétra-substitué par un ou des radicaux hydroxyalkyle en C₂-C₃, et NH(R₇)ₙ(R₈)ₘ⁺, R₇ étant un groupe alkyle en C₁-C₄, et R₈ étant un groupe hydroxyalkyle en C₂-C₃, les deux indices n et m étant des entiers valant 1 ou 2, et n # m ;
ainsi que leurs mélanges,
pour le traitement de surface de structures formées de feuilles cellulosiques.

2. Utilisation selon la revendication 1, pour laquelle la cellulose micronisée, et le ou les azurants fluorescents, sont utilisés dans une préparation, en particulier une préparation aqueuse.

3. Utilisation selon la revendication 2, pour laquelle la préparation est une liqueur de presse encolleuse ou une liqueur de presse applicatrice.

4. Utilisation selon la revendication 2, pour laquelle la préparation est respectivement un lait de couchage et une sauce de couchage.

5. Utilisation selon l'une quelconque des revendications précédentes, pour laquelle, pour la cellulose micronisée, la longueur moyenne des fibres et la granulométrie moyenne sont respectivement comprises dans une plage de 0,005 *µ*m à 15 *µ*m, et de préférence le diamètre maximal des fibres et la granulométrie maximale ne dépassent respectivement pas 20 *µ*m.

6. Utilisation selon l'une quelconque des revendications précédentes, pour laquelle, dans la formule (1) du ou des agents azurants fluorescents, un groupe de R₁ et R₂ est H, et l'autre groupe est choisi parmi SO₃⁻ et COOH.

7. Utilisation selon l'une quelconque des revendications précédentes, pour laquelle la structure formée de feuilles cellulosiques est un papier ou un carton.

8. Utilisation selon l'une quelconque des revendications 1, 2, 5 ou 6, pour laquelle la structure formée de feuilles cellulosiques est un matériau textile, en particulier un tissu de coton.

9. Préparation pour traitement de blanchiment en surface de structures contenant de la cellulose formée en feuilles, la préparation comprenant une cellulose micronisée ayant une longueur moyenne des fibres et une granulométrie moyenne respectivement inférieures ou égales à 15 *µ*m, ayant un diamètre maximal des fibres et une granulométrie maximale ne dépassant respectivement pas 25 *µ*m, et étant essentiellement exempte de fibres ayant une longueur dépassant 100 *µ*m, et au moins un agent azurant fluorescent (FWA) ayant la formule (1) 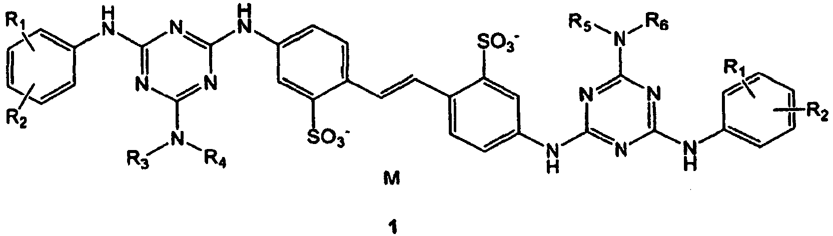 dans laquelle
R₁ et R₂ représentent chacun indépendamment de l'autre H, SO₃⁻ ou COOH, à la condition que, si R₁ est COOH, R₂ soit H, et inversement ;
R₃ et R₅ représentent chacun indépendamment de l'autre H, un groupe alkyle en C₁-C₄, hydroxyalkyle en C₂-C₃, CH₂COO⁻, CH₂CH₂CN, CH2CH₂CONH₂ ou CH₂CH₂SO₃⁻ ;
R₄ et R₆ représentent chacun indépendamment de l'autre H, un groupe alkyle en C₁-C₄, hydroxyalkyle en C₂-C₃, CH₂COO⁻, CH₂CH₂SO₃⁻, CH(COO⁻) CH₂COO⁻, CH(COO⁻) CH₂CH₂COO⁻, ou CH₂-phényle ; ou
R₃ et R₄, ou R₅ et R₆, indépendamment les uns des autres, forment ensemble avec l'atome d'azote un noyau morpholine ; et dans laquelle
M représente l'équivalent de charge correspondant choisi parmi H⁺, un cation de métal alcalin, ammonium, tétra(alkyle en C₁-C₄)ammonium, ammonium mono-, di-, tri-ou tétra-substitué par un ou des radicaux hydroxyalkyle en C₂-C₃, et NH(R₇)ₙ(R₈)ₘ⁺, R₇ étant un groupe alkyle en C₁-C₄, et R₈ étant un groupe hydroxyalkyle en C₂-C₃, les deux indices n et m étant des entiers valant 1 ou 2, et n # m.

10. Préparation selon la revendication 9, dans laquelle, rapportée en poids, la quantité de cellulose micronisée est inférieure ou égale à dix fois celle de l'agent azurant fluorescent.

11. Préparation selon la revendication 9, dans laquelle le rapport en poids de l'agent azurant fluorescent à la cellulose micronisée est compris dans la plage de 7:93 à 50:50 % en poids, de préférence de 10:90 à 50:50 % en poids, exprimé par la teneur en matière active de la préparation.

12. Préparation selon l'une quelconque des revendications 9 à 11, la préparation comprenant en outre de l'amidon, et étant une liqueur de presse encolleuse ou une liqueur de presse applicatrice.

13. Préparation selon l'une quelconque des revendications 9 à 11, la préparation comprenant en outre un pigment blanc et étant respectivement un lait de couchage et une sauce de couchage.

14. Procédé de traitement de blanchiment en surface de structures formées de feuilles cellulosiques, comprenant le traitement de la structure formée de feuilles cellulosiques avec une préparation telle que définie dans l'une quelconque des revendications 9 à 13.

15. Papier pouvant être obtenu par le procédé selon la revendication 14.
